# EUROPÄISCHE PATENTSCHRIFT

(11) **EP 0 607 822 B1**
(45) Veröffentlichungstag und Bekanntmachung des Hinweises auf die Patenterteilung: **16.10.1996**
(21) Anmeldenummer: 94100197.6
(22) Anmeldetag: 07.01.1994
(51) Int. Cl.: A61K 49/00

(54) **Flüssiggasformulierung zur Vitalitätsprüfung von Zähnen**
Formulation for testing the vitality of teeth containing liquid propellants
Formulation pour tester la vitalité des dents comprenant des gaz propulseurs liquifiés

(30) Priorität: 19.01.1993 DE 4301205
(43) Veröffentlichungstag der Anmeldung: 27.07.1994
(73) Patentinhaber: HOECHST AKTIENGESELLSCHAFT, 65929 Frankfurt am Main (DE)
(72) Erfinder: Deger, Hans-Matthias, Dr., D-65719 Hofheim/Ts (DE); Schütz, Claudia, D-65439 Flörsheim am Main (DE)

(56) Entgegenhaltungen:
- EP-A- 0 550 031
- WO-A-91/11173
- WO-A-91/16390
- WO-A-92/17558
- STOMATOL. DDR Bd. 38, Nr. 5 , 1988 Seiten 298 - 300 E.GLOCKMANN ET AL. 'Sensibilitätsprüfung der Pulpa mit dem Kältemittel F12'

## Beschreibung

Im Rahmen von zahnärztlichen Befunderhebungen und der Behandlungsplanung ist die Frage der Vitalität von Zähnen von entscheidender Bedeutung. Häufigste Ursache von Zahnschmerzen sind kariöse Defekte, Hyperämien der Pulpa (Markorgan des Zahnes), eine Pulpitis oder Gangrän. Da die Behandlung bei den verschiedenen Erkrankungen unterschiedlich ist, kommt der Pulpendiagnostik (Prüfung der Vitälität) eine entscheidende Bedeutung Zu. Bei tiefer Karies ist es wichtig, eine noch vorhandene Vitalität zu erhalten (z.B. durch direkte oder indirekte Überkappung). Bei Überkronungen ist es wichtig, devitale Zähne zu ermitteln, da devitale Seitenzähne eine überhöhte Frakturgefahr aufweisen und devitale Frontzähne vor der Überkronung mit einem Kernaufbau aus Metall zu versehen sind. Häufig treten auch Beschwerden bei überkronten Zähnen auf. Da hier die Diagnose durch Röntgenaufnahmen erschwert ist, kommt der Vitalitätsprüfung große Bedeutung zu.

Die Pulpa bildet als Reaktion auf äußere Einflüsse und Reize Dentin neu und erhält damit die Vitalität des Pulpengewebes.

Die Vitalität der Zahnpulpa ist gleichbedeutend mit dem intakten Stoffwechsel der Zellen und der Fähigkeit der Dentinneubildung. Eine direkte Beurteilung dieser Funktion ist nicht möglich, d.h. eine Überprüfung der Vitalität kann nur indirekt erfolgen.

In der Praxis wird die Vitalität der Pulpen durch Überprüfung der Sensibilität beurteilt. Eine etablierte klinische Methode ist die Anwendung von thermischen Reizen. Hierbei werden diese Reize an der Zahnoberfläche gesetzt und führen über eine Verschiebung des Dentinliquors zu einer Erregung der sensiblen Nerven der Pulpa und damit zu einer Schmerzempfindung.

Die Prüfung der Pulpensensibilität wird überwiegend mit Kältereizen durchgeführt. Diese Prüfung gilt als sicher, da die Reizschwelle für Kältereize im Pulpengewebe niedrig ist. Andererseits ist das Pulpengewebe in den meisten Fällen gegen (kurzzeitige) Abkühlung relativ unempfindlich, so daß keine bleibenden Gewebeschädigungen zu befürchten sind.

Hervorragend geeignet und seit Jahren erprobt für diese Prüfung ist der Einsatz von R12 (Dichlordifluormethan), wie in Stomatol. DDR, Band 38, Heft 5, 1988, Seiten 298-300 beschrieben.
Zur Prüfung der Vitalität eines Zahns wird dieser mit einem Schaumstoffpellet oder Wattebausch in Kontakt gebracht, der vorher mit druckverflüssigtem R12 besprüht worden war. Durch den Temperaturabfall an der Zahnoberfläche sinkt die Temperatur im Bereich der Pulpa, wodurch die Reizschwelle für Kältereize überschritten wird. R12 weist aber, wie alle vollhalogenierten Fluorchlorkohlenwasserstoffe (FCKW), eine hohe Beständigkeit gegen biotischen und abiotischen Abbau ab. FCKW werden in der unteren Atmosphäre nicht zerstört und steigen daher bis zur Stratosphäre auf. Es wird davon ausgegangen, daß sie dann durch UV-Strahlung gespalten werden und, über Radikale, zum Abbau der Ozonmoleküle beitragen.

Es wurde nun gefunden, daß R227 (1,1,1,2,3,3,3-Heptafluorpropan, 2-Hydroheptafluorpropan) alleine oder im Gemisch mit bis zu 50 Gew.-% R134a (1,1,1,2-Tetrafluorethan) als Ersatz für R12 geeignet ist. R227 und R134a sind beide unbrennbar. Da diese Verbindungen im Gegensatz zu vollhalogenierten Fluorchlorkohlenwasserstoffen Wasserstoff im Molekül enthalten, ist ihre atmosphärische Lebensdauer und damit ihr Beitrag zum indirekten Treibhauseffekt deutlich reduziert. Da sie chlorfrei sind, tragen sie nicht zum Ozonabbau bei. Dies bedeutet, daß der Einfluß auf die Atmosphäre minimiert wird.

Aus WO-A-9 116 390 sind azeotropartige Mischungen bekannt, die R134a, R152a (1,1-Difluorethan) sowie mindestens einen weiteren Halogenkohlenwasserstoff wie R227 enthalten. Eine Verwendung zur Vitalitätsprüfung von Zähnen wird nicht beschrieben.

Ein Gegenstand der vorliegenden Erfindung ist die Verwendung von R227 oder einer Mischung aus R227 und R134a, die mindestens 50 Gew.-% R227 enthält, zur Herstellung einer Flüssiggasformulierung zur Vitalitätsprüfung von Zähnen.

Übliche Spraydosen aus Aluminium sind auf einen Prüfdruck von 18 bar ausgelegt. Aus Sicherheitsgründen dürfen sie aber nur mit Produkten befüllt werden, deren Dampfdruck bei 50 °C maximal 12 bar beträgt, d.h. 2/3 des Prüfdrucks.
R227 hat bei 50 °C lediglich einen Dampfdruck von 9,22 bar. Dagegen beträgt der entsprechende Druck bei R134a bereits 12,77 bar. Daher kann R134a nur im Gemisch mit mindestens 50 Gew.-% R227 als "Drucksenker" eingesetzt werden. Der Dampfdruck eines Gemisches aus 50 Gew.-% R134a und 50 Gew.-% R227 beträgt 10,8 bar bei 50 °C, d.h. auch bei Befüllung der Spraydosen unter einem Luftdruck von 1 bar beträgt der Gesamtdruck in den Dosen nur 11,8 bar, hat also noch einen zulässigen Wert.

Ein weiterer Gegenstand der Erfindung ist daher eine Flüssiggasformulierung zur Vitalitätsprüfung von Zähnen, die 50 - 99 Gew.-% druckverflüssigtes R227 enthält, wobei der Rest im wesentlichen aus druckverflüssigtem R134a besteht. Vorzugsweise enthält die Flüssiggasformulierung 50 - 95 Gew.-% R227, vor allem 50 - 90 Gew.-% R227 insbesondere 50 - 70 Gew.% R227, wobei der Rest jeweils im wesentlichen aus R134a besteht.

Vergleichsuntersuchungen zeigen, daß R12, auf einen mit Watte umwickelten Meßfühler (Thermoelement) appliziert, innerhalb von 20 - 25 Sekunden die Temperatur auf -36 °C senkt, d.h. etwa 10 °C unter den Siedepunkt von R12. Überraschenderweise wurde nun gefunden, daß R227 die Temperatur innerhalb von 15 Sekunden nur auf etwa -16 °C senkt, also bis etwa zum Siedepunkt von R227. Ein Gemisch aus je 50 Gew.-% R227 und R134a erreicht innerhalb von 15 Sekunden eine Temperatur von -26,5 °C, was auch nur etwa dem Siedepunkt dieses Gemisches entspricht. Dabei tritt sowohl bei reinem R227 als auch bei seinem Gemisch mit R134a eine wesentlich geringere Vereisung am Meßfühler auf als bei Einsatz von R12.
Diese Untersuchungen lassen erwarten, daß R227 und dessen Mischungen mit maximal 50 Gew.-% R134a auf der Zahnoberfläche (über einen Wattebausch oder Schaumstoffpellet appliziert oder direkt auf den Zahn gesprüht) eine Senkung der Temperatur bewirken wird, die zur Überschreitung der Schmerzgrenze eines vitalen Zahns ausreicht.
R227 und seine genannten Mischungen mit R134a ermöglichen daher eine schonendere aber ebenso wirksame Vitalitätsprüfung von Zähnen wie das bisher für diesen Zweck eingesetzte R12. Es werden durch die höhere Temperatur, im Vergleich zu R12, Schäden am Zahnschmelz und dem umgebenden Gewebe verhindert oder minimiert und ebenso die Auswirkungen beim versehentlichen Aufsprühen auf die Schleimhaut.

Mit R227 und seinen Mischungen mit R134a kann sich der Arzt an die individuelle Schmerzgrenze des Patienten anpassen, indem er, beginnend mit reinem R227, den Anteil an R134a langsam erhöht und damit die Temperatur am Zahn langsam senkt. Je höher der Anteil an R134a ist, umso tiefer liegt die am Zahn erzeugte Temperatur. Das heißt, es kann eine wesentlich schonendere Diagnose erfolgen als mit R12.
Mit reinem R227 oder mit geringe Mengen an R134a enthaltendem R227 kann die Vitalitätsprüfung auch durch direktes Aufsprühen auf den Zahn vorgenommen werden. Jedoch ist es vorzuziehen, genauso wie bei Mischungen mit höheren Anteilen an R134a, eine Applikationshilfe (z.B. Wattebausch oder Schaumstoffpellet) zu verwenden.

Insgesamt haben R227 und seine Mischungen mit R134a folgende Vorteile gegenüber R12: Sie sind chlorfrei und tragen daher nicht zum Ozonabbau bei. Ihr Dampfdruck ist so niedrig, daß keine Befüllung der Spraydosen im Vakuum notwendig ist. Sie sind wegen der weniger tiefen von ihnen am Zahn erzeugte Temperatur schonender anzuwenden. Durch geeignete Wahl des R134a-Anteils kann die Vitalitätsprüfung an die individuelle Schmerzgrenze des Patienten angepaßt werden.
Dabei können Mischungen mit steigenden Anteilen von R134a eingesetzt werden, beginnend mit 1 - 5 Gew.-%, dann 5 - 10 Gew.-%, dann 10-20 Gew.-%, dann 20 - 30 Gew.-%, dann 30 - 40 Gew.-%, dann 40-50 Gew.-% R134a, wobei der Rest jeweils im wesentlichen R227 ist.

Die folgenden Beispiele erläutern die Erfindung.

### Vergleichsbeispiel

Ein Thermoelement wurde an ein digitales Temperatur-Anzeigegerät angeschlossen. Das Thermoelement war mit einem Wattepellet (3 cm hoch und 4-5 mm dick) umwickelt. Eine mit R12 gefüllte Druckgasdose wurde an einem Stativ fixiert. Der Abstand des Sprühkopfs vom Thermoelement betrug 10 cm; das Thermoelement war dabei zentral im Wattepellet justiert. Bei Raumtemperatur wurde ca. 5 Sekunden lang R12 auf den Wattepellet gesprüht. Innerhalb von 20 - 25 Sekunden, ab dem Beginn des Sprühens gerechnet, sank die Temperatur auf -36 °C. Dabei trat eine starke Vereisung am Wattepellet auf.

### Beispiel 1

Es wurde wie im Vergleichsbeispiel gearbeitet, nur daß statt R12 jetzt R227 auf den Wattepellet gesprüht wurde. Innerhalb von 15 Sekunden, ab dem Beginn des Sprühens gerechnet, sank die Temperatur auf -16 °C, wobei nur eine schwache Vereisung am Wattepellet auftrat.

### Beispiel 2

Es wurde wie im Vergleichsbeispiel gearbeitet, nur daß statt R12 jetzt eine Mischung aus 50 Gew.-% R227 und 50 Gew.-% R134a auf den Wattepellet gesprüht wurde. Innerhalb von 15 Sekunden, ab dem Beginn des Sprühens gerechnet, sank die Temperatur auf -26,5 °C, wobei nur eine mäßige Vereisung am Wattepellet auftrat.

### Beispiel 3

Es wurde wie im Vergleichsbeispiel gearbeitet, nur daß statt R12 jetzt eine Mischung aus 90 Gew.-% R227 und 10 Gew.-% R134a auf den Wattepellet gesprüht wurde. Innerhalb von 15 Sekunden, ab dem Beginn des Sprühens gerechnet, sank die Temperatur auf -20 °C, wobei nur eine schwache Vereisung am Wattepellet auftrat.

## Patentansprüche

1. Flüssiggasformulierung zur Vitalitätsprüfung von Zähnen, die 50-99 Gew.-% druckverflüssigtes 2-Hydroheptafluorpropan enthält und wobei der Rest im wesentlichen aus druckverflüssigtem 1,1,1,2-Tetrafluorethan besteht.

2. Flüssiggasformulierung nach Anspruch 1, dadurch gekennzeichnet, daß sie 50-95 Gew.-% 2-Hydroheptafluorpropan enthält und der Rest im wesentlichen aus 1,1,1,2-Tetrafluorethan besteht.

3. Flüssiggasformulierung nach Anspruch 1, dadurch gekennzeichnet, daß sie 50-90 Gew.-% 2-Hydroheptafluorpropan enthält und der Rest im wesentlichen aus 1,1,1,2-Tetrafluorethan besteht.

4. Flüssiggasformulierung nach Anspruch 1, dadurch gekennzeichnet, daß sie 50-70 Gew.-% 2-Hydroheptafluorpropan enthält und der Rest im wesentlichen aus 1,1,1,2-Tetrafluorethan besteht

5. Verwendung von 2-Hydroheptafluorpropan zur Herstellung einer Flüssiggasformulierung für die Vitalitätsprüfung von Zähnen.

6. Verwendung einer Mischung aus 2-Hydroheptafluorpropan und 1,1,1,2-Tetrafluorethan, die mindestens 50 Gew.-% 2-Hydroheptafluorpropan enthält, zur Herstellung einer Flüssiggasformulierung für die Vitalitätsprüfung von Zähnen.

7. Verwendung einer Mischung nach Anspruch 6, dadurch gekennzeichnet, daß sie 50-99 Gew.-% 2-Hydroheptafluorpropan enthält und der Rest im wesentlichen aus 1,1,1,2-Tetrafluorethan besteht.

8. Verwendung einer Mischung nach Anspruch 6, dadurch gekennzeichnet, daß sie 50-95 Gew.-% 2-Hydroheptafluorpropan enthält und der Rest im wesentlichen aus 1,1,1,2-Tetrafluorethan besteht

9. Verwendung einer Mischung nach Anspruch 6, dadurch gekennzeichnet, daß sie 50-90 Gew.-% 2-Hydroheptafluorpropan enthält und der Rest im wesentlichen aus 1,1,1,2-Tetrafluorethan besteht

10. Verwendung einer Mischung nach Anspruch 6, dadurch gekennzeichnet, daß sie 50-70 Gew.-% 2-Hydroheptafluorpropan enthält und der Rest im wesentlichen aus 1,1,1,2-Tetrafluorethan besteht

## Claims

1. A liquefied gas formulation for testing the vitality of teeth, which comprises 50-99% by weight of 2-hydroheptafluoropropane liquefied under pressure, the remainder essentially being 1,1,1,2-tetrafluoroethane liquefied under pressure.

2. A liquefied gas formulation as claimed in claim 1, which comprises 50-95% by weight of 2-hydroheptafluoropropane, the remainder essentially being 1,1,1,2-tetrafluoroethane.

3. A liquefied gas formulation as claimed in claim 1, which comprises 50-90% by weight of 2-hydroheptafluoropropane, the remainder essentially being 1,1,1,2-tetrafluoroethane.

4. A liquefied gas formulation as claimed in claim 1, which comprises 50-70% by weight of 2-hydroheptafluoropropane, the remainder essentially being 1,1,1,2-tetrafluoroethane.

5. The use of 2-hydroheptafluoropropane for the preparation of a liquefied gas formulation for testing the vitality of teeth.

6. The use of a mixture of 2-hydroheptafluoropropane and 1,1,1,2-tetrafluoroethane, which comprises at least 50% by weight of 2-hydroheptafluoropropane, for the preparation of a liquefied gas formulation for testing the vitality of teeth.

7. The use of a mixture as claimed in claim 6, which comprises 50-99% by weight of 2-hydroheptafluoropropane, the remainder essentially being 1,1,1,2-tetrafluoroethane.

8. The use of a mixture as claimed in claim 6, which comprises 50-95% by weight of 2-hydroheptafluoropropane, the remainder essentially being 1,1,1,2-tetrafluoroethane.

9. The use of a mixture as claimed in claim 6, which comprises 50-90% by weight of 2-hydroheptafluoropropane, the remainder essentially being 1,1,1,2-tetrafluoroethane.

10. The use of a mixture as claimed in claim 6, which comprises 50-70% by weight of 2-hydroheptafluoropropane, the remainder essentially being 1,1,1,2-tetrafluoroethane.

## Revendications

1. Formulation de gaz liquéfié pour le contrôle de vitalité des dents, qui contient de 50 à 99 % en poids de 2-hydroheptafluoropropane liquéfié sous pression, le reste étant constitué essentiellement de 1,1,1,2-tétrafluoroéthane liquéfié sous pression.

2. Formulation de gaz liquéfié selon la revendication 1, caractérisée en ce qu'elle contient de 50 à 95 % en poids de 2-hydroheptafluoropropane, le reste étant constitué pour l'essentiel de 1,1,1,2-tétrafluoroéthane.

3. Formulation de gaz liquéfié selon la revendication 1, caractérisée en ce qu'elle contient de 50 à 90 % en poids de 2-hydroheptafluoropropane, le reste étant constitué pour l'essentiel de 1,1,1,2-tétrafluoroéthane.

4. Formulation de gaz liquéfié selon la revendication 1, caractérisée en ce qu'elle contient de 50 à 70 % en poids de 2-hydroheptafluoropropane, le reste étant constitué pour l'essentiel de 1,1,1,2-tétrafluoroéthane.

5. Utilisation du 2-hydroheptafluoropropane pour préparer une formulation de gaz liquéfié destinée au contrôle de vitalité des dents.

6. Utilisation d'un mélange de 2-hydroheptafluoropropane et de 1,1,1,2-tétrafluoroéthane, qui contient au moins 50 % en poids de 2-hydroheptafluoropropane, pour préparer une formulation de gaz liquéfié destinée au contrôle de vitalité des dents.

7. Utilisation d'un mélange selon la revendication 6, caractérisée en ce qu'il contient de 50 à 99 % en poids de 2-hydroheptafluoropropane, le reste étant constitué pour l'essentiel de 1,1,1,2-tétrafluoroéthane.

8. Utilisation d'un mélange selon la revendication 6, caractérisée en ce qu'il contient de 50 à 95 % en poids de 2-hydroheptafluoropropane, le reste étant constitué pour l'essentiel de 1,1,1,2-tétrafluoroéthane.

9. Utilisation d'un mélange selon la revendication 6, caractérisée en ce qu'il contient de 50 à 90 % en poids de 2-hydroheptafluoropropane, le reste étant constitué pour l'essentiel de 1,1,1,2-tétrafluoroéthane.

10. Utilisation d'un mélange selon la revendication 6, caractérisée en ce qu'il contient de 50 à 70 % en poids de 2-hydroheptafluoropropane, le reste étant constitué pour l'essentiel de 1,1,1,2-tétrafluoroéthane.
